Europäisches Patentamt

European Patent Office

Office européen des brevets

(11) Publication number: **0 153 063**
A2

# EUROPEAN PATENT APPLICATION

(21) Application number: 85300703.7

(22) Date of filing: 01.02.85

(51) Int. Cl.⁴: **C 07 K 15/06**, A 61 K 35/26, A 61 K 39/395

(30) Priority: 03.02.84 US 576945
01.06.84 US 616159
18.01.85 US 692552

(43) Date of publication of application: 28.08.85
Bulletin 85/35

(84) Designated Contracting States: **AT BE CH DE FR GB IT LI LU NL SE**

(71) Applicant: **VXR, Inc., 217 Read Street, Portland Maine 04103 (US)**

(72) Inventor: **Goldfarb, Marcia F., 9 Boldoin Street, Portland Maine 04102 (US)**

(74) Representative: **De Minvielle-Devaux, Ian Benedict Peter et al, CARPMAELS & RANSFORD 43, Bloomsbury Square, London WC1A 2RA (GB)**

(54) Inducer factor of T-suppressor cells.

(57) A biologically active composition isolated from vertebrate tissue, capable of inducing immature bone marrow cells to differentiate into competent suppressor T-cells, comprising a factor which is heat stable to approximately 80°C, has a pl range of 6.6-7.2, a molecular weight range of 45,000 to 70,000 daltons as calculated by gel filtration, a molecular weight range of 47,000 to 52,000 daltons as calculated by two-dimensional electrophoresis, and is essentially free of serum albumin.

–1–

INDUCER FACTOR OF T-SUPPRESSOR CELLS

### Field of the Invention

This invention relates to a new composition of matter, and more particularly to a novel thymic factor isolated in purified form from mammalian thymus tissue which induces immature bone marrow cells to differentiate into competent suppressor T-cells (Ts). This novel thymic factor, which can also be called inducer of T-suppressor cells (Ts inducer factor), has a molecular weight range of 45,000-70,000 daltons as determined by gel filtration, a pI of 6.6-7.2 as determined with an ampholine electrofocussing column, and is heat stable up to about 80°C. Ts inducer factor induces immature bone marrow cells to differentiate into cells which exhibit a phenotypic marker of suppressor T-cells and which when adoptively transferred repress reconstitution of the immune response in irradiated, thymectomized mice. Antibodies have been raised to Ts inducer factor. This invention also relates to therapeutic methods and fields of use for Ts inducer factor.

### Background of the Invention

The essential role of the thymus gland in the development of immunological competence in animals and man is now generally accepted. In thymectomized animals thymus grafts can restore immunological competence. Findings of this nature have led to the discovery of many different thymic factors. Most of these factors are derived from the thymus gland but some are obtained from serum or thymus epithelial cell cultures. For reviews and general discussion on the nature of thymic factors and how they function in the development of the immune response, see, inter alia, Tada, T. et al Adv. Immunology 28, 1 (1979); Golub, E. S., The Cellular Basis of the Immune Response, 2d Ed., Sinauer Associates, Massachusetts 1981, chapter 19; White, A. Ann. N.Y. Acad. Sci. 332, 23 (1979); and Kruisbeeck, A. Thymus 1 163 (1979).

The mammalian immune system consists of a sophisticated complex of interactive cells and cell products. The immune system develops in the fetus and neonatally, just after birth. Lymphocyte progenitor cells, derived from pluripotential hemopoietic stem cells, first appear in the yolk sac of the developing embryo and in the fetal liver. After birth they are found in the bone marrow, where they persist for life. These lymphocyte progenitor cells are capable of irreversibly differentiating into either of two classes of immunologically competent cells: T lymphocytes or B lymphocytes. Both classes of lymphocytes are ubiquitous in the blood, lymph, spleen and lymph nodes. T and B lymphocytes appear to be morphologically similar - they are generally small, motile, nonphagocytic cells - yet they bear different immunogenic markers on their plasma membranes and perform distinctly different immunological functions.

T lymphocytes (hereinafter referred to as T-cells) develop inside the thymus gland from lymphocyte progenitor cells which have migrated through the bloodstream from the bone marrow. Factors with hormone-like effect produced inside the thymus apparently induce the progenitor cells to differentiate into functional T-cells with protective or regulatory capacities. Following a 2 to 5 day maturation process, the T-cells leave the thymus and enter the general blood and lymph circulation.

Some of the progenitor lymphocytes become "helper" T-cells (Th), that is, they interact with the other class of lymphocytes (B-cells) to cause them to mature, divide and become antibody-producing clones. Another subclass of T-cells produced by the thymus is the "suppressor" population (Ts). These cells internally regulate the immune system so that only the type and amount of antibody or protective T cell is produced that is needed. When this suppressor system is not regulated, certain forms of arthritis or autoimmune disease can occur. Yet another class of lymphocytes that arise in the bone marrow are the so-called "natural killer" cells. These white cells eliminate body cells that have become infected with viruses and other microbes, and they also destroy defective cells such as cancers.

The differentiation of these distinct types of immunologically competent T-cells is controlled by an as yet incompletely understood constellation of thymic factors.

The other class of lymphocytes, called B lymphocytes or , B-cells, apparently differentiate inside the bone marrow, liver and spleen from lymphocyte progenitor cells. Then these cells circulate in the bloodstream. The immunological response of mature B cells may be regulated by T-cells or T-cell products. Mature B cells synthesize and secrete immunoglobulin antibodies in response to antigenic stimulation. For many antigens, B-cells require the presence of T-helper cells before they can produce antibodies. The mechanism of this T-B cooperation is poorly understood, but it is recognized to be inhibited by T-suppressor cells.

The present invention encompasses a thymic factor which causes immature bone marrow cells to become suppressor T-cells. This factor is herein designated inducer of T-suppressor cells, or Ts inducer factor. This invention also embraces a novel method of isolating the suppressor inducer factor of this invention.

Mammalian bone marrow cells, when incubated with this Ts inducer factor , become competent suppressor T-cells. It is understood that this capacity of Ts inducer factor to induce the effective agents of immunosuppression can be used for therapeutic purposes. For example, human bone marrow cells can be transformed into suppressor T-cells for injection into transplant patients to cause in vivo suppression of foreign tissue rejection. It is also contemplated that pharmaceutical preparations of Ts inducer factor can be prepared for direct in vivo use under a variety of conditions. Naturally occurring autoimmune diseases, which have been correlated with a loss of suppressor cells, may be susceptible to treatment with Ts inducer factor. Such autoimmune diseases include systemic lupus erythematosus, hemolytic anemia, multiple sclerosis, severe ectopic eczema, hyper-IgE syndrome, and inflammatory bowel disease. See Reinherz, E. L. et al, Immunology Today (April, 1981) pp. 69-75. Allergies are also thought to derive from defects in the suppressor T-cell system; hence it is contemplated that Ts inducer factor can be of therapeutic use in controlling allergies. And, since loss of the suppressor T-cell population may correlate temporarily with the severity of other clinical diseases, it is further contemplated that Ts inducer factor can be used as an adjuvant medication of general utility.

During the past decade a multiplicity of factors which exhibit hormonal activity have been isolated from the mammalian thymus gland. These thymic factors form a remarkably diverse assemblage with respect to the methodologies of their extraction, states of purification, physicochemical properties, and biological activities. Several of these thymic factors have been reported to induce some sort of suppression of the immune system: e.g., anti-thymosin, Thymopoietin I and Thymopoietin II, THF, thymosin $\alpha_7$, FTS, and SDIP. All of these reported thymic suppressor factors have molecular weights that are an order of magnitude lower than that of Ts inducer factor.

Great Britain Patent Specification No. 1,195,980, published June 24, 1970 described hormone-like preparations derived from thymus gland produced at Yeshiva University, N.Y. The in vivo injection of one such preparation, called anti-thymosin, was reported to inhibit lymphoid tissue proliferation and induce a decrease in the number of blood lymphocytes. This anti-thymosin appeared to have the properties of a protein of molecular weight less than 5000.

U.S. Patent No. 4,120,951, issued October 17, 1978 to Goldstein and assigned to Sloan-Kettering Institute for Cancer Research, discloses two closely related polypeptides, designated Thymopoietin I and Thymopoietin II, from bovine thymus. The Goldstein patent states that these polypeptides can be used to inhibit the uncontrolled proliferation of thymin-responsive lymphocytes. The molecular weights of Thymopoietin I and II were reported to be around 6,000 to 7,000 daltons.

Kook et al in 1975 reported the isolation of THF, a thymic hormone of molecular weight 3220 and isoelectric point 5.66-5.90. Kook, A. I. et al, Cellular Immunology 19:151 (1975). Shohat et al recently described the induction of T suppressor cells by in vitro treatment of lymphocytes of renal allograph recipients with THF. Shohat, B. et al, Transplantation 35(1):68 (1983).

Ahmed et al reported that thymosin $\alpha_7$ acts on prothymocytes to induce suppressor cells. Ahmed, A. et al, Ann. N.Y. Acad. Sci. 332:81 (1979). Thymosin $\alpha_7$ reportedly has a molecular weight of 2,200 daltons and a pI of around 3.5. Low, T. L. K., et al, Ann. N.Y. Acad. Sci. 332:32 (1979).

Facteur thymique serique (FTS) is a peptide of molecular weight close to 900 that has been isolated from both thymus tissue and normal serum. Bach, J. F., in Advances in Pharmacology and Therapeutics, Vol. 4 (Pergamon Press, 1979) p. 145. FTS is reported to activate suppressor T cells in various in vivo and in vitro systems, especially when administered at high pharmalogical dosages. It is reportedly not known whether FTS stimulates mature suppressor cells or induces a maturation of suppressor T cell precursors. Bach, J. F., in Cell Lineage, Stem Cells and Cell Determination: INSERM Symposium No. 10, p. 261 (N. LeDouarin, Ed., Elsevier/North-Holland Biomedical Press, 1979).

A spleen-derived immunosuppressive peptide (SDIP) has recently been reported to have physicochemical properties and enzymatic susceptibilities similar to those of the thymic hormone FTS, supra. When injected into sheep erythrocyte (SRBC)-sensitized mice, at the last step of differentiation of the lymphocytes, SDIP reportedly reduced the plaque-forming capacity of spleen cells from the treated animals; a similar inhibitory response was observed with FTS. Lenfant, M. et al, Immunology 48:635 (1983).

U.S. Patent No. 4,239,498, issued to Rule on December 16, 1980 discloses and claims thymic factors prepared according to a sequential acetone fractionation process. Rule disclosed a suppressor which reportedly repressed reconstitution of the immune response in lethally irradiated thymectomized mice. No physicochemical characterization of this apparently crude thymic extract was presented.

Another suppressor factor has very recently been reported. See Kasakura, S., et al, The Journal of Immunology 130(6):2720 (June, 1983). The reported physical description of this factor — molecular weight of 18,000-29,000 daltons, pI of 6.2-7.3, and heat resistance only to 56°C — eliminates the possibility of it being our Ts inducer factor.

In addition to the above-mentioned thymic suppressor factors, the administration of theophylline has also been reported to activate suppressor T cells from peripheral human blood. Shapira demonstrated a lack of suppressor T-cells in patients with acute rejection episodes (ARE) following kidney transplants. After administration of theophylline-ethyl diamine

-8-

(aminophyline), the ARE in 12 of 16 patients was abrogated and suppressor T-cells reappeared in their peripheral blood. Shapira, Z. Transplantation Proceedings 14(1):113 (1982).

It will be noted that the thymic suppressor factors in the prior art discussed above are of comparatively lower molecular weight than Ts inducer factor, which has a molecular weight range of 45,000 to 70,000 daltons. Indeed, a review of the prior art indicates that most of the thymic factors which have been categorized have molecular weights of less than 10,000 daltons. See, e.g., Goldstein, A. L. et al, in Recent Progress in Hormone Research, Vol. 37, p. 369 (Greeb R. O., Ed., Academic Press, 1981). Furthermore, the lack of homology among those small factors which have been sequenced suggests that they are not cleavage products of a common precursor. Trainin, N., et al, Immunology Today 4(1):16 (1983).

The few high molecular-weight thymic factors which have been described can be distinguished from Ts inducer factor by their biological activities, which tend to enhance rather than suppress immunological competence.

For example, Mizutani et al extracted two purified hypocalcemic proteins, designated $TP_1$ and $TP_2$, from bovine thymus gland. The molecular weight of $TP_1$ was found to be 68,000 daltons and that of $TP_2$ to be 57,000 daltons. Both are heat labile (56°C, 30 min.). Significant increases in antibody-producing cells were found, as indicated by increases in plaque forming cells, when either $TP_1$ or $TP_2$ was injected into neonatal mice. Mizutani et al, Chem. Pharm. Bull. 25(9):2156 (1977).

White et al obtained a homogeneous protein with thymic hormone-like activity from blood serum. Its molecular weight was determined to be 56,700 $\pm$ 300 daltons, and its physical, chemical, and immunological properties indicated an identity with authentic human prealbumin. The biological activities of this high molecular weight compound included inducing an increase in the numbers of sheep erythrocyte plaque-forming cells (IgM) in vitro by spleen cells from neonatally thymectomized mice treated in vivo with the purified compound. White et al, Annals New York Academy of Sciences 332:1 (1979). The use of human serum prealbumin for increasing immunological competence was the subject of U.S. Patent No. 4,046,877, issued to White et al on September 6, 1977 and assigned to Syntex (U.S.A.) Inc. The data presented in the patent's Example 3 indicates that human prealbumin significantly increased the capacity of spleen cells to synthesize IgM and IgG antibodies, as evidenced by increased numbers of plaque-forming cells.

Pierschbacher et al reported the extraction of a lymphocyte stimulating hormone, denoted LSHr, from beef thymus. LSHr reportedly is a peptide with a molecular weight of 80,000 and an isoelectric point of about 4.55. Administration of microgram quantities of LSHr to nude mice for 2-3 weeks induced development of T-cell function as determined by antibody response to a T-dependent antigen and a response of spleen cells to T-cell mitogen. Ann. N.Y. Acad. Sci. 332:49 (1979).

Other high molecular-weight thymic factors have been reported by Jin et al, who reportedly extracted a mixture of polypeptides, of molecular weights 9000-68,000 daltons and

0153063

-10-

isoelectric points 5.0-7. , from pork thymus. Said factors reportedly increase rosette formation in fetal thymocytes, which indicates generally the induction of T-cell differentiation. Jin, T. et al J. Nanking University 1:115 (1979), cited in Goldstein et al, Recent Progress in Hormone Research, Vol. 37, supra, at p. 381.

The methods employed to extract the above-mentioned factors from thymus tissue typically consist of two general steps. First, a crude extract is prepared from thymus gland — typically by homogenization, heat treatment, centrifugation and filtration. Second, the factor or factors in the crude extract are isolated by enrichment procedures — typically by dialysis, molecular sieve chromatography, affinity chromatography, and/or preparative electrophoresis.

The extraction procedure of the present invention, as will be discussed in greater detail below, also involves a two-part procedure. A crude thymus extract is first prepared in conformance with procedures well known in the art. Said crude extract is then subjected to a novel and surprisingly effective enrichment protocol.

The crude thymic extract, as used at the start of this inventive process, is prepared in general conformity with the methods described by others; see, e.g., A. Goldstein's Thymic Fraction 2, in U.S. Patent No. 4,010,148. The crude thymic extract is fractionated by molecular sieve chromatography and contaminants are removed by affinity chromatography using immunoadsorbents.

## Detailed Description of the Invention

The novel thymic factor Ts inducer factor of the present invention is isolated from mammalian thymus tissue, especially calf thymus. Neonatal vertebrate thymus tissues other than calf thymus are also suitable for the isolation of this factor, but are usually somewhat less available than calf thymus tissue.

The Ts inducer factor can be isolated by means of the following general procedure. An extract of mammalian thymus is prepared. Said thymus extract is chromatographed by gel filtration on Sephadex G-75 and pooled according to size. The appropriate size pool (MW=70,000-25,000) is absorbed by solid phase affinity chromatography using an anti-bovine serum albumin column. The unabsorbed fraction is rechromatographed using an anti-bovine serum column. The unabsorbed fraction is chromatographed by gel filtration on Sephadex G-75 and pooled according to size. The appropriate size pool (MW=70,000-50,000) is tested for the presence of Ts inducer factor by activity assays with murine bone marrow cells.

For example, a crude extract of mammalian thymus tissue is first prepared. Thymus tissue is obtained from freshly slaughtered neonatal vertebrates, preferably from two-week to two-month old calves. It was discovered that the freshly collected thymus glands cannot be frozen for future use without impairing the activity of the Ts inducer product.

It is preferred procedure to remove extraneous connective and adipose tissues that adhere to the thymus glands. The glands are then minced. It was found convenient to homogenize the minced thymus glands in approximately 200 g aliquots.

The minced glands are placed in a two-fold (v/w) excess of homogenization buffer. A suitable buffer for this purpose is 10 mM Tris-HCl, pH 8.0, containing 50 mM NaCl and 0.5 mM $MgCl_2$. The thymus glands are homogenized in the aforementioned buffered saline at high speed for approximately 2 minutes. A suitable homogenization apparatus is a Waring blender. Said homogenization and many of the subsequent steps are carried out at 4°C in order to slow down protease and other enzymatic activity, and also to inhibit bacterial contamination. An antibiotic was not added because it might affect the subsequent bioassays.

The resulting homogenate is centrifuged for approximately 30 minutes at 12,000 x g at 4°C, or until the solid cellular debris has precipitated. The aqueous supernatant is recovered by conventional techniques for further processing. It was observed that any fats on top of the aqueous supernatant will come out later, after heating and the subsequent centrifugation; therefore special care need not be taken to remove them at this point.

The recovered aqueous supernatant is heated to approximately 80°C for 30 to 45 minutes or until the material has congealed. This congealed material consists of denatured proteins, including proteases which otherwise inactivate the Ts inducer factor. The congealed solids are then removed by centrifugation for approximately 30 minutes at 12,000 x g at 4°C. The aqueous supernatant is collected for further processing; the precipitated pellet is discarded.

The aqueous supernatant is then frozen to -80°C. This is also a convenient storage point in the extraction process.

It was observed that said aqueous supernatant can be stored at -80°C for at least 2-3 years without significant impairment of the Ts inducer factor activity. It was also found convenient to freeze said aqueous supernatant in 200 ml aliquots, as subsequent purification steps are conveniently carried out with this size of sample.

After thawing at 37°C, e.g. in a water bath, the extract is centrifuged for approximately 30 minutes at 45,000 x g at 4°C. This high speed centrifugation serves to remove those marginally soluble proteins which were denatured further by freezing and any particulate matter not removed during low speed centrifugation. The resulting supernatant is then concentrated approximately 16-fold by ultrafiltration; e.g., in an Amicon stirred cell fitted with a Y-10 membrane in order to eliminate small molecules of less than, e.g., 10,000 dalton molecular weight. The filtrate containing the low-molecular-weight molecules is discarded.

Those familiar with the field of art to which this invention contributes a novel and unobvious advancement will recognize the above-mentioned preliminary preparatory steps as not departing in significant detail from the protocol of A. Goldstein and others, except that fresh, unfrozen thymus tissue is used as starting material. However, from this point on the method of the present invention departs from the prior art in novel and unobvious ways. The crude extract collected by the above-mentioned preparatory steps is fractionated by two molecular sieve chromatography passages and further refined by absorptions on an anti-bovine serum albumin column and an anti-bovine serum column.

First, the concentrated supernatant from the above-mentioned ultrafiltration step is centrifuged for 15 minutes at 45,000 x g at 4°C in order to remove any remaining solid material which might occlude the gel filtration column. Any pellet formed at this point is discarded. The supernatant is collected and subjected to molecular sieve chromatography in order to selectively isolate molecules within the 70,000 to 25,000 dalton size range. The preferred method of accomplishing said size fractionation is by gel filtration on Sephadex G-75 (Source: Pharmacia) at 6.8 cm x $hr^{-1}$, using phosphate buffered saline, pH 7.2, without $NaN_3$. Biogel (Bio-Rad) or Ultragel (LKB) may also be suitably employed to isolate molecules within the specified size range. The 70,000 to 25,000 dalton fractions are pooled and concentrated by ultrafiltration, e.g., in an Amicon stirred cell fitted with a YM-10 membrane, which will pass molecules having molecular weights less than 10,000 daltons.

The 70,000 to 25,000 dalton concentrate is centrifuged for approximately 10 minutes at 1,500 x g, in order to remove any proteins which sheared during the concentration step. Any precipitate is discarded. The recovered supernatant is then subjected to affinity chromatography in which rabbit antiserum to bovine serum albumin (BSA) is used as an immunoadsorbent. This immunoadsorbent was selected after it was discovered that Ts inducer factor has a molecular weight range similar to that of the contaminating BSA. Sources of rabbit anti-BSA include Miles Laboratories, Elkhart, Indiana. Suitable solid substrata to which rabbit anti-BSA can be bonded include Sepharose-6B (Pharmacia). The antibody is bound to the solid substratum by means of cyanogen bromide activation of the gel. Passage of the supernatant from the above-mentioned centrifugation step over the anti-BSA column will result in the physical removal, by adsorption, of contaminating BSA molecules.

The unadsorbed fraction is then subjected to further purification by affinity chromatography in which rabbit antiserum to bovine whole serum (BS) (Source: Miles Laboratories) is used as the immunoadsorbent. It was found that the sequence of anti-BSA followed by anti-BS is a convenient and efficient method. Albumen is the major contaminant, and other proteins are minor contaminants. Removing them first allows the second size fractionation to be accomplished more easily.

The recovered supernatant is subjected to further size fractionation in order to isolate only a 70,000 to 50,000 dalton fraction. This can also be accomplished by gel filtration chromatography on Sephadex G-75 at 3.6 cm x hr$^{-1}$, using sodium azide-free phosphate buffered saline, pH 7.2. The 70,000 to 50,000 dalton fractions are pooled and then concentrated by ultrafiltration, e.g., in an Amicon stirred cell fitted with a YM-10 membrane.

The 70,000 to 50,000 dalton fraction concentrate is centrifuged at approximately 1,500 x g for 10 minutes. Any precipitate is discarded. The resulting supernatant is tested for the presence and concentration of Ts inducer factor. Such testing can be accomplished by a combination of physicochemical and biological assays. Physicochemical assays will provide information as to the concentration and degree of homogeneity of the isolated suppressor inducer factor. Suitable physicochemical assays to accomplish this purpose include determinaton of molecular weight by, e.g., electrophoresis; pI determination by isoelectric focusing on, e.g., agarose or polyacrylamide gel; and determination of protein content by reading optical density at 280 nm.

Bioassays suitable for confirming the presence of the Ts inducer factor include assessments of the induction of suppressor T-cell differentiation by, e.g., phenotypic assay for surface antigens characteristic of suppressor T-cells, or by functional assay for suppressor T-cell activity.

The following examples are presented to illustrate the invention but it is not to be considered as limited thereto.

## Example 1

The preferred method for isolating and purifying the iTs factor is by means of the following procedure.

1. Thymus glands obtained from freshly slaughtered calves were minced following the removal of connective tissue and other extraneous tissue. The glands were placed in a two-fold (v/w) excess of homogenization buffer (10 mM Tris-HCl, pH 8.0, containing 50 mM NaCl and 0.5 mM $MgCl_2$). The thymus glands were homogenized in a Waring blender for two minutes at maximum output at 4°C, and the resulting homogenate was centrifuged for 30 minutes at 12,000 x g at 4°C. The supernatant was heated to approximately 80°C for 30 to 45 minutes or until the material had congealed. The solids were removed by centrifugation for 30 minutes at 12,000 x g at 4°C. The supernatant was frozen at -80°C in 200 ml aliquots. After thawing at 37°C, the extract was centrifuged for 30 minutes at 45,000 x g at 4°C. The supernatant was concentrated 16-fold in an Amicon stirred cell fitted with a YM-10 membrane.

2. The concentrated supernatant from step 1 was centrifuged for 15 minutes at 45,000 x g at 4°C. Any precipitate removed at this point was discarded. The supernatant was fractionated by gel filtration on Sephadex G-75 at 6.8 cm x hr$^{-1}$, using phosphate-buffered saline, pH 7.2, without NaN$_3$. The 70,000 to 25,000 dalton fraction was pooled and concentrated in an Amicon stirred cell fitted with a YM-10 membrane.

3. The 70,000 to 25,000 dalton concentrate was centrifuged for 1C minutes at 1,500 x g. Any precipitate was discarded. The supernatant was subjected to affinity chromatography in which rabbit antiserum to bovine serum albumin was used as the immunoadsorbent.

4. The unadsorbed fraction was further fractionated by affinity chromatography in which rabbit antiserum to bovine whole serum was used as the immunoadsorbent.

5. The unabsorbed fraction from the previous step was subjected to further purificaton by gel filtration chroma-tography on Sephadex G-75 at 3.6 cm x hr$^{-1}$, using phosphate buffered saline, pH 7.2, without NaN$_3$. The 70,000 to 50,000 dalton fraction was pooled and concentrated in an Amicon stirred cell fitted with a YM-10 membrane.

6. The 70,000 to 50,000 dalton fraction concentrate was centrifuged at 1,500 x g for 10 minutes. Any precipitate formed at this step was discarded.

-18-

7. The supernatant from the preceding step was tested for protein content, characterized by electrophoretic techniques, and tested by bioassay for the presence of the Ts inducer factor.

The Ts inducer factor isolated in Example 1 was partially characterized with reference to physicochemical properties, e.g., protein content, molecular weight, and electrophoretic activity.

Protein content was established by measurement of optical density at 280 nm (ISCO UA-5 monitor with a Type 6 optical unit).

The molecular weight of the Ts inducer factor was estimated by two methods, gel filtration and two-dimensional electrophoresis.

Gel filtration was accomplished as described in Gel Filtration: Theory and Practice (Pharmacia Fine Chemicals). A Pharmacia column (2.6 x 98 cm) packed with Sepahdex G-75-120 and equilibrated with phosphate-buffered saline, pH 7.2, without $NaN_3$, was used. The column was run with an upward flow which was maintained with a Technicon Auto Analyzer proportioning pump. A molecular weight range of 45,000 to 60,000 daltons was obtained by gel filtration of the Ts inducer factor isolated in Example 1.

Two-dimensional electrophoresis was also accomplished as described in Operation of the ISO-DALT System (Tollaksen, Anderson and Anderson, Argonne National Laboratories, Argonne, Illinois, 1981). By this method, the Ts inducer factor isolated in Example 1 was found to have a molecular weight range of approximately 47,000 to 59,000 daltons.

An isoelectric point (pI) range of 6.6-7.2 was ascertained using an 8100-Ampholine Electrofocussing column as described in LKB Instrument Manual 1-8100ED4.

The biological activity of the Ts inducer factor isolated in Example 1 was ascertained by means of a phenotypic assay for suppressor T-cells. The Ts inducer factor was tested for the ability to cause immature mouse prethymocytes from bone marrow to differentiate into suppressor T-cells. The suppressor T-cells were detected by means of a phenotypic assay in which diagnostic surface antigens are detected by antigen-specific monoclonal antibodies.

The Ly2 surface antigen is thought to be a marker for identification of mouse suppressor T-cells, whereas the Ly1 surface antigen is thought to be a marker for mouse helper T-cells. See Golub, E. S., The Cellular Basis of the Immune Response (pp. 262-265, Sinauer Associates, Inc., Sunderland, MA, 1981).

The capacity of Ts inducer factor to induce suppressor T-cell differentiation was ascertained by the following procedure. Bone marrow cells derived from 9-12 week old BALB/c female mice (Jackson Laboratories, Bar Harbor, ME) were incubated with aliquots of the Ts inducer factor isolated in Example 1. Bone marrow cells were prepared by the method of Schmeige, S., and H. Miller, Journal of Immunology 113:110 (1974). Approximately $10^7$ bone marrow cells were suspended in 1.0 ml of RPMI 1640 + 5% FBS; an aliquot of Ts inducer factor was added; and the sample was incubated for four hours at 37°C. Samples containing Ts inducer factor at concentrations ranging from 0.05 to 50 µg/ml were prepared in duplicate sets. Duplicate sets of controls were also prepared, consisting of spleen cells, thymus cells, and bone marrow cells — all without Ts inducer factor. The control cells were obtained from syngeneic mice as described in Journal of Immunology 113:110 (1974).

After incubation the cells were washed with fresh medium; the cells were centrifuged at approximately 1,000 x g then resuspended in 5 ml of RPMI 1640 + 5% FBS. One set of the samples and controls was treated with monoclonal antibody to Ly2 surface antigen; while the replicate set was treated with monoclonal antibody to LY1 surface antigen. (Source of monoclonal antibodies: Becton Dickinson, Monoclonal Antibody Center, Sunnyvale, CA). Said monoclonal antibodies were used at a 1:1000 dilution. Each sample and control was incubated for one hour at 4°C in the presence of monoclonal antibody.

The cells were then washed with fresh medium, and biotinylated horse antiserum to mouse IgG (Source: Miles Laboratories, Cappel Laboratories, Downington, PA) was added. The samples were incubated for an additional hour with the biotinylated antiserum, washed with fresh medium, and treated with fluorisothiocyanate (FITC)-conjugated avidin (Victor Laboratories, Burlingame, CA).

After reacting with FITC-conjugated avidin, the samples were washed and resuspended in fresh medium. Each sample was counted on a cytofluorometer (Cytofluorograph 50, Ortho Diagnostics) and the percentage of cells expressing the LY1 or LY2 surface antigens was recorded. The results of this assay are presented in Table I.

---

TABLE I

In vitro assay of Ts inducer factor for induction of Ly2 surface antigen:

|  | Ly1 | Ly2 |
|---|---|---|
| 50 µg/ml | 5% | 21% |
| 10 µg/ml | 2% | 24% |
| 1 µg/ml | 0% | 17% |
| 0.5 µg/ml | 0% | 8% |
| 0.05 µg/ml | 0% | 0% |
| Control, spleen cells | 12% | 8% |
| Control, thymus cells | 80% | 92% |
| Control, bone marrow cells | 3% | 5% |

Material tested is representative of the Ts inducer factor isolated by the process described in Example 1.

Concentrations are given for amount of Ts inducer factor incubated per $10^7$ mouse bone marrow cells.

Percentages indicate the number of cells expressing the Ly1 or Ly2 surface antigens, which are thought to identify mouse helper or suppressor T-cells, respectively.

---

The maximum expression of Ly2 or Ly1 in bone marrow cells is approximately 23% in this assay. Such maximum expressions, based on empirical observation, are considered to be due to inherent limitations on the number (approximately 23%) of bone marrow cells which are capable of being induced to become functional suppressor or helper T-cells. The higher values observed in the thymic cell controls are considered to be due to their a priori differentiation within the thymus gland.

The biological activity of the Ts inducer factor isolated in Example 1 was further ascertained by means of a functional assay for suppressor T-cells. The Ts inducer factor was tested for the ability to cause immature mouse prethymocyte cells from bone marrow to differentiate into suppressor T-cells. Here the suppressor T-cells were detected by their ability to suppress the murine immune system.

BALB/c female mice were used as syngeneic cell donors and recipients throughout the procedure of this assay. The procedures used to extract, prepare, and incubate the mouse bone marrow cells were identical to those employed in the phenotypic assay, supra.

Approximately $10^7$ mouse bone marrow cells were incubated in culture media (RPMI 1640 + 5% FBS) with various aliquots of Ts inducer factor for 4 hours at 37°. Controls consisted of mouse bone marrow cells ($10^7$) incubated with bovine serum albumin in place of Ts inducer factor.

After incubation, the cells were washed three times in culture media. Approximately $10^7$ of the washed cells were injected i.v. into thymectomized-irradiated (900R) mice. These mice also received by injection either $10^9$ sheep red blood cells (SRBC) to induce suppressor T-cells, or $10^6$ SRBC to induce helper T-cells. See Journal of Immunology 17:313 (1976).

T-cells were obtained for control purposes by adoptively transferring $10^7$ murine thymus cells of the same strain into thymectomized-irradiated (900R) syngeneic recipient mice. These control mice were also injected i.v. with either $10^9$ or $10^6$ SRBC to induce T-suppressor or T-helper cells, respectively. Five to seven mice were included in each group.

Two or four days later (for T-helper or T-suppressor cells, respectively), $10^6$ spleen cells from said experimental and control adoptive transferee mice were prepared, by the method of H. Miller et al, Journal of Immunology 115:839 (1975). Said suspensions of spleen cells were assigned to $10^7$ normal spleen cell cultures. These were set up according to the method of Mishell and Dutton, as described in Selected Methods in Immunology, B. B. Mishell and S. M. Shiigi, eds., Freedman, San Francisco, 1980, pp. 31-37 and 72-77.

The antibody-producing cells were indicated by clear plaques representing immunologically lysed SRBC. The results of this assay are presented in Table II. The incidence of statistically fewer plaques, or antibody-producing cells, is considered to indicate the suppression of B-cell activity.

---

## TABLE II

In vitro assay of Ts inducer factor for suppression of antibody production:

|  | T-Helper ($10^6$SRBC) | T-Suppressor ($10^9$SRBC) |
|---|---|---|
| 50 µg/ml | 527 | 148 |
| 10 µg/ml | 810 | 143 |
| 1 µg/ml | 1955 | 235 |
| 0.5 µg/ml | 3090 | 3715 |
| 0.05 µg/ml | 3000 | 3000 |
| Control, 200 µg/ml BSA | 3170 | 3250 |
| Control, thymus cells | 7230 | 230 |

Material tested is representative of the Ts inducer factor isolated by the process described in Example 1.

Concentrations are given for amount of Ts inducer factor incubated per $10^7$ mouse bone marrow cells.

Numbers indicate plaques formed.

---

The premise of this bioassay is that pretreatment of pluripotential bone marrow cells with Ts inducer factor will induce their differentiation into competent T-suppressor cells. If so, when said treated bone marrow cells are injected, along with SRBC which serve as antigen templates for antibody formation, into mice whose immune systems have been destroyed, the suppressor T-cells induced by the composition of this invention should act to repress antibody formation to the SRBC antigens. Such immunosuppression is demonstrable _in_ _vitro_ by a relative decrease in plaque forming cells. Note that such immunosuppression is shown here: At concentrations greater than 0.5 µg/ml the Ts inducer factor reduces the incidence of plaque formation to below that of the maximum number of control thymus cells which are capable of forming plaques. The thymus control cells injected i.v. along with $10^9$ SRBC are considered to represent a baseline equivalent to a mature suppressor system.

### Example 2

Antibodies to Ts inducer factor were prepared by the following procedure: Rabbits were injected subcutaneously with an emulsion of Ts inducer factor and complete Freund's adjuvant (1:1). After one month, secondary injections with Ts inducer factor and incomplete Freund's adjuvant were given subcutaneously. The animals were bled after 9-11 more days.

Subsequent boosts (secondary injections) and bleeds were carried out at 14-18 days and 9-11 days, respectively. Antibodies specific for the Ts inducer factor were isolated from serum by conventional means well known to those versed in the serological art.

Said isolated antibodies were shown to be specific for Ts inducer factor in the following manner. The antibodies were bound to an affinity chromatography column, using materials and methods identical to those described in Example 1, and used as an immunoadsorbent to separate their specific binding partners from a crude thymic extract, prepared as in Example 1. Bound molecules were eluted with 4 M $MgCl_2$. The isolated molecules were assayed by physicochemical assays and two bioassays, exactly as described in Example 1. The results of those assays indicated that the Ts inducer factor had been isolated from crude thymic extract by means of this preparatory process.

Example 3

Monoclonal antibody to Ts inducer factor are produced, selected and cultured by known methods; see, e.g., Hurrell, J. G. R., Monoclonal Hybridoma Antibodies: Techniques and Applications, CRC Press, Boca Raton, Florida, 1982.

Example 4

The antibodies prepared according to either Example 2 or 3 are used in conjunction with Ts inducer factor as diagnostic markers for immunoregulatory abnormalities. Serum specimens are treated with specific antibody to detect abnormal levels of Ts inducer factor. Such abnormal levels are indicative of immune system disorders. High levels of Ts inducer factor, as determined by the use of the antibody, are indicative of immune deficiency, where as low levels of Ts inducer factor suggest hypersensitivity conditions such as allergies or autoimmune diseases.

### Example 5

The antibodies prepared according to Example 2 or 3 are used therapeutically to regulate the immune system in cases of mammalian immune deficiency diseases, especially those characterized by abnormally low blood levels of T helper cells. To minimize the differentiation and maturation of T suppressor cells and induce a higher ratio of helper to suppressor cells, specific antibody to Ts inducer factor is papain-modified, and fragments are purified by Sephadex gel filtration and the thus purified antibodies are administered. This may be effected intraperitoneally, especially in some mammalian species, or by other means. In humans, e.g., it may be advantageous to culture the specific antibody in vitro with a protein of the patient's bone marrow and then substitute the so treated marrow culture for untreated diseased marrow via a marrow transplant procedure. Other methods of administration will readily occur to those skilled in the art. By reducing the rate of formation and relative blood level of T suppressor cells, this antibody treatment enables improved immune response.

### Example 6

In another therapeutic application, the Ts inducer factor isolated according to Example 1 or Example 2 is used to therapeutically suppress the immune system of an allograft recipient. Bone marrow cells from the potential transplant recipient are incubated in vitro with Ts inducer factor to maximize the induction of T suppressor cells. Said induced Ts cells are then returned to the transplant recipient, where they suppress an immunological reaction to the new tissue.

### Example 7

Balb/c mice were hyperimmunized with AKR mouse brains by injection of 1 cu. mm. brains intraperitoneally every 7 days for 30 days. The mice were found to display expected and evident neuropathy at 30 days, including inability to move toward food or another mouse, tremors and general disorientation. The mice were tested for anti-thy 1.1 antibody (see Miller and Kaufman, Clin. Immunol. Immunopath. 7, 288-299 (1977)) at day 30 and were separated into 8 groups of 20 mice each. Of these groups, one was not treated; three were each treated intraperitoneally every 3 days thereafter with thymic suppressor inducer factor prepared as in Example 1 at respective levels per group of 1 µg per injection, 10 µg per injection and 100 µg per injection. The four remaining groups were treated intravenously on day 30 with marrow cells: one group with $10^6$ cells pretreated with 1 µg TsIF, one group with $10^7$ cells with 1 µg TsIF, another with $5 \times 10^7$ cells pretreated with 1 µg TsIF and the last with $5 \times 10^7$ untreated cells. The following table shows the anti-Thy-1.1 titer of each group at day 30 and the time to death up to a maximum of 120 days, of each group.

## Table 3

| Mice injected with 1 mm AKR brain (IP) every 7 day | Titer anti-Thy-1.1 (day 30) | Time to death (days) |
|---|---|---|
| TsIF 1 µg/day IP | f 1:10,000 | 43-48 |
| TsIF 10 µg/day IP | 1:2,000 | 90-111 |
| TsIF 100 µg/day IP | 1:500 | 90 (50% still living) |
| No TsIF | > 1:10,000 | 40-47 |
| $10^6$ TsIF treated marrow cells | 1:1000 | 83 (10% still living) |
| $10^7$ TsIF treated marrow cells | 1:200 | all living |
| $5 \times 10^7$ treated marrow cells | 1:400 | all living |
| $5 \times 10^7$ untreated marrow cells | > 1:10,000 | 51-97 |

## Example 8

The experiment of Example 7 was modified by usig Balb/c mice immunized by intraperitoneal injection of 1 cu mm. of Balb/c mouse brain every 7 days, whereupon after 30 days all showed marked signs of neuropathy, including essential disability as described in Example 7. Anti-brain titer was detected by fluorescent assay on fixed brain tissue on day 30. The mice were separated into 4 groups of 5 individuals each and one group was not treated. Of the remaining groups, one received 1 µg daily of TsIF prepared as per Example 1 from day 30 to day 60 and the other groups, respectively, received 10 µg and 100 µg every 3 days (from day 30 to day 60) of the same TsIF. TsIF was administered by intraperitoneal injection in all cases.

Results are shown in table 4.

Table 4

| Mice injected with 1 mm$^3$ syngeneic brain (IP) every 7 days | Titer anti-brain (day 30) and evidence of neuropathy | Time to reversal to neuropathy |
|---|---|---|
| TsIF 1$^\mu$g/day IP | 1:1000 +++ | No change at 60 days (+++) |
| TsIF 10$^\mu$g/day IP | 1:250 +++ | Mice healthy at 60 days (+) |
| TsIF 100$^\mu$g/day IP | 1:100 +++ | Mice healthy at 60 days (+) |
| No TsIF | 1:100 +++ | No change at 60 days (+++) |

Key:  +++ = Disabled
       ++ = Tremors
        + = Slightly awkward, near normal in appearance

In a continuation of this experiment B cells and T cells were harvested from spleens of sacrificed mice from the groups of mice involved in Table 4 and from untreated mice. The cells were partially concentrated by passage through nylon wool columns (B cells were retained and mechanically removed; T cells passed through). Further Balb/c Mice were again autoimmunized as described for the Table 4 portion of the experiment, separated into 7 groups of 5 mice each and tested for anti-brain titer at 30 days, when all showed marked evidence of neuropathy. One group was not treated; of the other six groups, one received normal Balb/c B cells (b), one received normal Balb/c T cells (T), one received autoimmunized Balb/c B cells (B$^1$), one received autoimmunized Balb/c T cells (T$^1$); one TSIF-treated Balb/c B cells (B*) and one TsIF-treated Balb/c T cells (T*). The dosages in each instance were 3 x $10^6$ cells per mouse injected intravenously at day 30.

The results appear in table 5:

Table 5

| Cell Population injected at day 30 (3 x $10^6$ cells I.V.) | Titer anti-brain and evidence of neuropathy at day 30 postinfection |
|---|---|
| 1. B | 1:2000 +++ |
| 2. T | 1:2500 +++ |
| 3. B' | 1:3500 +++ |
| 4. T' | 1:1000 ++ |
| 5. B* | 1:750  ++ |
| 6. T* | 1:100  + . |
| 7. - | 1:1000 +++ |

+++ Disabled
++ Tremors
+ Slightly awkward

## Example 9

Natural MRL/L p r mice (Jackson Laboratories), which are genetic strains that develop rheumatoid arthritis and lupus erythmatosus naturally, were treated at 12 weeks of age with

(a) intraperitoneally injected TsIF prepared as in Example 1 at dosage level 10 µg every 3 days;

(b) intraperitoneally injected TsIF prepared as in Example 1 at dosage level 50 µg every 3 days;

(c) a single intravenous injection of TsIF pretreated bone marrow cells (3 x $10^6$).

A control group of these mice (20 individuals) was maintained.

-31-

After 30 days into the treatment period, none of the treated mice had died, but 80% of the controls had died corresponding to the mean death time of 22 weeks for this strain of mice.

As used herein, "TsIF" means the novel thymic suppressor inducer factor produced as in Example 1.

While the invention has been described with particular reference to experiments on lower mammals, those skilled in the art will understand that it is useful in the diagnosis and therapy of mammals generally including humans. Other embodiments and uses will readily occur to those skilled in the art on reading the foregoing, and it is accordingly not intended to limit the present invention except as required by the appended claims.

0153063

CLAIMS

1.  A biologically active composition isolated from vertebrate tissue, capable of inducing immature bone marrow cells to differentiate into competent suppressor T-cells, comprising a factor which is heat stable to approximately 80°C, has a pI range of 6.6-7.2, a molecular weight range of 45,0 00 to 70,000 daltons as calculated by gel filtration, a molecular weight range of 47,000 to 52,000 daltons as calculated by two-dimensional electrophoresis, and is essentially free of serum albumin.

2.  A composition as in Claim 1, isolated from mammalian thymus tissue.

3.  A composition as in Claim 1, isolated from tissue cell cultures of thymic epithelial cells.

4.  A composition as in Claim 1 or Claim 3, isolated from tissue cell cultures of bovine thymic epithelial cells.

5.  A process for isolating a suppressor inducer factor composition from thymus tissue, the steps of which comprise:

    A.   preparing a crude thymus extract;

    B.   fractionating said crude thymus extract by molecular sieve chromatography to isolate a 70,000 to 25,000 dalton fraction;

    C.   contacting said 70,000 to 25,000 dalton fraction with an immunoadsorbent containing antibodies to bovine serum albumin;

    D.   contacting the unadsorbed material from step C with an immunoadsorbent containing antibodies to bovine whole serum;

    E.   fractionating the unadsorbed material from step D by molecular sieve chromatography to isolate a 70,000 to 50,000 dalton fraction; and

    F.   collecting the 70,000 to 50,000 fraction from step E.

6. A process as in claim 5, in which the immunoadsorbents in steps C and D are in solid phase.

7. A process as in claim 5, in which the immunoadsorbents in steps C and D are in liquid phase.

8. The process of any one of claims 5 to 7, in which the crude thymus extract of step A is prepared as follows:

A. removing connective and adipose tissue from thymus glands of freshly slaughtered neonatal vertebrates;

B. homogenizing said thymus glands in a buffered solution;

C. centrifuging the homogenate to remove cellular debris;

D. heating the supernatant from step C to approximately 80°C until soluble proteins, including proteases, have congealed;

E. removing said congealed material by centrifugation;

F. freezing the supernatant from step E;

G. thawing the supernatant and removing any solids by high speed centrifugation;

H. concentrating the supernatant from step G by ultra-filtration using a membrane that retains molecules larger than 10,000 daltons.

9. A process for extracting a suppressor inducer factor composition from thymus tissue, the steps of which comprise:

A. raising antibodies specific to the suppressor inducer factor composition of any one of claims 1 to 4 of the composition isolated by the process of any one of claims 5 to 8;

B. bonding said specific antibodies to a gel chromatography column to form an immunoadsorbent;

C. passing a crude thymic extract over said immunoabsorbent;

D. washing away unbound materials; and

E. eluting the bound suppressor inducer factor composition from the immunoadsorbent.

0153063

10. The process of any one of claims 5 to 9 wherein the antibodies are of monoclonal origin.

11. The process of any one of claims 5 to 9, wherein the antibodies are of polyclonal origin

12. A therapeutic composition comprising a therapeutically effective amount of the composition of any one of claims 1 to 4 or the composition isolated by the process of any one of claims 5 to 11 and, as necessary, an appropriate pharmaceutical vehicle.

13. A composition according to claim 12 containing 10 to 100 µg of the composition.

14. A polyclonal or monoclonal antibody to the suppressor inducer composition of any one of claims 1 to 4 or the composition isolated by the process of any one of claims 5 to 11.

15. A method of diagnosis wherein the antibody of claim 14 is used to detect the level of Ts inducer factor in a sample of blood taken from a patient.

16. The composition of any one of claims 1 to 4, the therapeutic composition of claim 12 or claim 13, the composition isolated by the process of any one of claims 5 to 11, or the antibody of claim 14, for use in a therapeutic method.